Europäisches Patentamt

European Patent Office

Office européen des brevets

(19)

(11) Veröffentlichungsnummer: **0 044 388**
**A2**

(12) # EUROPÄISCHE PATENTANMELDUNG

(21) Anmeldenummer: 81103962.7

(22) Anmeldetag: 23.05.81

(51) Int. Cl.³: **G 01 N 21/59**
**G 01 N 33/48**

(30) Priorität: 21.07.80 CH 5564/80

(43) Veröffentlichungstag der Anmeldung:
27.01.82 Patentblatt 82/4

(84) Benannte Vertragsstaaten:
AT BE CH DE FR GB IT LI NL

(71) Anmelder: F. HOFFMANN-LA ROCHE & CO.
Aktiengesellschaft

CH-4002 Basel(CH)

(72) Erfinder: Eisenwiener, Hans-Georg, Dr.
Kirschenstrasse 26
D-7858 Weil/Rhein(DE)

(74) Vertreter: Kloter, Rudolf et al,
Postfach 3255
Grenzacherstrasse 124 CH-4002 Basel(CH)

(54) Verfahren zur Bestimmung von einem oder mehreren Parametern in Proben.

(57) Es wird ein Verfahren für die Vereinfachung der Gehaltsermittlung bei Profil- und Einzelbestimmungen unter exakten analytischen Bedingungen und optimaler Kontrolle beschrieben.

Bei diesem Verfahren werden pro Parameter in ein und
derselben Messzelle und mit ein und demselben Reagens
mehrere gleiche Bestimmungen hintereinander und nur mit
einmaliger Pipettierung von Reagens durchgeführt.

EP 0 044 388 A2

F.Hoffmann-La Roche & Co.Aktiengesellschaft, Basel/Schweiz

RAN 4090/123

Verfahren zur Bestimmung von einem oder mehreren Parametern in Proben

Gegenstand der Erfindung ist ein Verfahren für die Vereinfachung der Gehaltsermittlung bei Profil- und Einzelbestimmungen unter exakten analytischen Bedingungen und optimaler Kontrolle.

In klinisch-chemischen Laboratorien werden die Analysen im allgemeinen serienmässig durchgeführt. Pro Serie von 10 bis 20 verschiedenen Proben-Ansätzen werden in getrennten Küvetten (Messzellen) ein Reagenzien-Leerwert-Ansatz, ein Standard-Ansatz für die Berechnung und ein Ansatz mit einem Kontrollserum von bekanntem Gehalt mitgeführt.

Aus den gemessenen Extinktionsdifferenzen der Proben-Ansätze und des Kontrollserum-Ansatzes werden mit Hilfe der gemessenen Extinktionen des Standard-Ansatzes die Gehalte in den Proben und im Kontrollserum berechnet.

Berechnungsformel:

$$\text{Gehalt der Probe} = \frac{\text{gemessene Extinktionsdifferenz der Probe}}{\text{gemessene Extinktionsdifferenz des Standards}} \times \text{Konzentration des Standards}$$

Klt/ 6.5. 1981

Der Gehalt im Kontrollserum ergibt sich nach der gleichen Formel, indem im Zähler die Extinktionsdifferenz der Probe durch diejenige des Kontrollserums ersetzt wird.

Bei einer Serie von 10 bis 20 Proben sind somit ein Reagenzien-Leerwert-Ansatz, ein Standard-Ansatz und ein Kontrollserum-Ansatz mitzuführen.

Wird nach Beginn bzw. Beendigung der Durchführung der jeweiligen Serienbestimmung eine zusätzliche Probe zur Gehaltsbestimmung ins Labor geschickt (z.B. Notfall), ist für diese eine Probe in getrennten Küvetten sowohl ein Reagenzien-Leerwert-Ansatz, ein Standard-Ansatz und Kontrollserum-Ansatz durchzuführen.

Bei einer einzigen Gehaltsbestimmung müssen ebenfalls in getrennten Küvetten ein Reagenzien-Leerwert-Ansatz, ein Standard-Ansatz und ein Kontrollserum-Ansatz mitgeführt werden, was Einzelbestimmungen wesentlich verteuert.

Ebenfalls ungünstig liegen die Verhältnisse bei der Durchführung von sogenannten Profilanalysen von einem Patienten. Ist z.B. von einem Patienten ein Notfallprofil anzufertigen, bedarf es der Durchführung von zahlreichen Analysen-, Standard-, Kontroll- und Reagenzien-Leerwert-Ansätzen. Besteht dieses Notfallprofil aus einer Glukose-Bestimmung (ev. diabetisches Koma), Harnstoffbestimmung (ev. urämisches Koma), GOT-, GPT- und CK-Bestimmung (ev. "koronares"-Ereignis) und wird diese Profilbestimmung mit einem Analysator exakt und unter der in einigen Ländern, z.B. der BRD, schon obligatorischen Qualitätskontrolle durchgeführt, bedarf es folgender 17 Ansätze:

|  | Probe | Ansatz für Reagentien-Leerwert | Standard | Kontroll-serum |
|---|---|---|---|---|
| Glucose | + | + | + | + |
| Harnstoff | + | + | + | + |
| GOT | + | + |  | + |
| GPT | + | + |  | + |
| CK | + | + |  | + |

Für diese Bestimmung werden also 17 mal Reagens und bei Verwendung von Wegwerfküvetten 17 Küvetten benötigt.

Im Rahmen der vorliegenden Erfindung wurde nun gefunden, dass es nicht erforderlich ist, für jeden Ansatz Reagens zu pipettieren und die einzelnen Ansätze in getrennten Küvetten durchzuführen. Es ist vielmehr möglich, in ein und derselben Küvette und mit nur einmaliger Pipettierung von Reagens den Reagenzien-Leerwert-, den Proben-, den Standard- und den Kontrollserum-Ansatz hintereinander auszuführen. Das Verfahren wird im folgenden Konsekutiv-Messverfahren genannt.

Gegenstand der Erfindung ist demnach ein Verfahren zur Bestimmung von einem oder mehreren Parametern in Proben, welches dadurch gekennzeichnet ist, dass in ein und derselben Messzelle und mit ein und demselben Reagens mehrere gleiche Bestimmungen hintereinander (konsekutiv) und mit nur einmaliger Pipettierung von Reagens durchgeführt werden.

Die einzelnen Bestimmungen, die mit demselben Reagens hintereinander durchgeführt werden, können mehrere verschiedene Proben- und/oder Standard-Ansätze, die in die Messzelle nacheinander eingeführt werden, betreffen. Es handelt sich hierbei also um eine Mehrfachbestimmung eines Parameters verschiedener Proben in einer Küvette.

Mit demselben Reagens können insbesondere hintereinander ein Reagenzien-Leerwertansatz, mindestens ein Probenansatz, ein Standard-Ansatz und ein Kontrollserum-Ansatz

- 4 -

bestimmt werden, welche nacheinander in die Messzelle eingeführt werden. Hierbei handelt es sich um die Bestimmung eines Parameters in einer Probe in einer Messzelle (Einzelbestimmung).

Mit dem erfindungsgemässen Verfahren kann aber auch ein Parameter in mehreren, verschiedenen Proben in mehreren Messzellen bestimmt werden (Batch-Konsekutiv-Bestimmung). Weiterhin erlaubt das erfindungsgemässe Verfahren die Bestimmung von mehreren Parametern, in mehreren gleichen Proben (Profilbestimmung). Das erfindungsgemässe Verfahren wird in seinen wesentlichen Aspekten im folgenden anhand der Bestimmung einer Enzymaktivität, einer Substratkonzentration und einer Profilbestimmung sowie den spezifischen Ausführungsbeispielen näher erläutert. Das erfindungsgemässe Verfahren eignet sich sowohl für manuelle Bestimmungen wie auch für Bestimmungen in einem Zentrifugalanalysator.

Bestimmung einer Enzymaktivität

Bei exakter Durchführung werden bei einer Einzelbestimmung drei Ansätze benötigt: Der Reagenzien-Leerwert-Ansatz, der Proben-Ansatz und der Kontrollserum-Ansatz. Dazu ist in drei Küvetten das Reagens zu pipettieren und anschliessend in die Reagenzien-Leerwert-Küvette eine dem Probenvolumen entsprechenden Menge an bidestilliertem Wasser, in die Proben-Küvette die Probe mit dem zu ermittelnden Gehalt und in die Kontrollserum-Küvette die entsprechende Menge an Kontrollprobe.

Nach der erfindungsgemässen Methode wird in die zu verwendende Küvette das Reagens pipettiert. Daran anschliessend werden die der jeweiligen Probenmenge entsprechende Menge an Wasser für den Regenzien-Leerwert pipettiert und der Messwert für den Reagenzien-Leerwert innerhalb der Messzeit T1 ermittelt. Anschliessend wird die Probe pipettiert und innerhalb der Messzeit T2 der Messwert für die Probe und den Reagenzien-Leerwert ermittelt. Daran anschliessend wird nun die Kontrollprobe pipettiert und innerhalb der Messzeit T3 die Summe der Messwerte für den Reagenzien-Leerwert, für die Probe und für die Kontrollprobe ermittelt.

Die Ermittlung der Aktivität in der unbekannten Probe erfolgt folgendermassen: Messwert 1 = Reagenzien-Leerwert

Messwert 2 = Reagenzien-Leerwert
+ Probe

Messwert 3 = Reagenzien-Leerwert
+ Probe
+ Kontrollprobe

Messwert 3 - Messwert 2 = Aktivität in der Kontrollprobe
Messwert 2 - Messwert 1 = Aktivität in der unbekannten
Probe

Selbstverständlich muss bei der Berechnung der Aktivitäten den veränderten Volumenverhältnissen (verschiedene Endvolumina bei Messwert 1, 2 und 3) Rechnung getragen werden.

Entspricht die Aktivität der Kontrollprobe dem bekannten (deklarierten Wert) dann wird die erfindungsgemässe Messung gesamthaft als richtig beurteilt und die Aktivität der unbekannten Probe als richtig angesehen.

Entspricht die Aktivität der Kontrollprobe nicht dem bekannten (deklarierten) Wert, dann wird die erfindungsgemässe Messung als nicht richtig angesehen und die Aktivität der unbekannten Probe in einem neuen Ansatz, z.B. mit einer verdünnten Probe, ermittelt. Da bei den heutigen allgemein optimierten Testkonzentrationen die Linearitätsbereiche für eine Gehaltsbestimmung sehr gross sind, und der Gehalt in der Kontrollprobe im Normalbereich liegt und für die Bestimmung des Regenzien-Leerwertes nahezu kein Substrat verbraucht wird, ist der Linearitätsbereich für die unbekannte, zu analysierende Probe bei den allermeisten Bestimmungen ausreichend.

Bestimmung einer <u>Substratkonzentration</u>

Um z.B. die Stoffwechsellage eines Diabetikers (hyperglykämisches bzw. hypoglykämisches Koma) durch Labordaten anhand seiner Glukose-Werte im Blut zu bestätigen, ist häufig im Notfall die Glukose-Bestimmung durchzuführen. Nach herkömmlicher Methode sind bei exakter Analytik vier Ansätze durchzuführen: 1. Reagenzien-Leerwert-Ansatz, 2. Ansatz für die Probe, deren Gehalt bestimmt werden soll, 3. Standard-Ansatz für die Berechnung und 4. Ansatz für das mitzuführende Kontrollserum.

Nach herkömmlicher Methode sind dazu notwendig: viermal Reagens und - bei Verwendung von Wegwerfküvetten - vier Küvetten. Wird diese Bestimmung nach der erfindungsgemässen Methode durchgeführt, werden nur einmal Reagens und eine einzige Küvettenposition benötigt.

Die Messung der Extinktionen und Pipettierung von Proben erfolgt gemäss folgendem Schema:

- 7 -

0044388

E = gemessene Extinktion

$t_1$ = Zeitpunkt der Pipettierung für den Reagentien-Leerwert

$t_2$ = Zeitpunkt der Pipettierung für die Probe

$t_3$ = Zeitpunkt der Pipettierung für den Standard

$t_4$ = Zeitpunkt der Pipettierung für das Kontrollserum

S = Standard

P = Probe

K = Kontrollserum

RL = Reagentien-Leerwert

Die Berechnung des Gehaltes in der unbekannten Probe erfolgt nach den bekannten Formeln:

$$\text{Berechnungsfaktor} = \frac{\text{Konzentration des Standards}}{\Delta E(S) - \Delta E(RL)} = F$$

$$\text{unbekannter Gehalt in der Probe} = F \times \left[\Delta E(P) - \Delta E(RL)\right]$$

$$\text{Gehalt in der Kontrollprobe} = F \times \left[\Delta E(K) - \Delta E(RL)\right]$$

Selbstverständlich muss bei den zur Berechnung verwendeten Extinktionen den veränderten Volumenverhältnissen Rechnung getragen werden.

Wenn der ermittelte Gehalt in der Kontrollprobe dem deklarierten Wert entspricht, wird der ermittelte Gehalt in der unbekannten Probe als richtig angesehen.

Profilbestimmung

Auf dem folgenden Schema geht das Prinzip der erfindungsgemässen Messung und Hinzupipettierung der Volumina an Wasser (für Reagenzien-Leerwert), Standard (für die Berechnung bei Substratbestimmungen), Probe - in welchen die Gehalte an GOT, GPT, CK, Harnstoff und Glukose ermittelt werden sollen - und des Kontrollserums hervor.

1. Messzyklus für Reagentien-Leerwert

2. Messzyklus

3. Messzyklus

|  | GOT | GPT | CK | Glukose | Harn-stoff |  |
|---|---|---|---|---|---|---|
| Zeit-punkt 0: | ✗ | ✗ | ✗ | ✗ | ✗ | Pipettierung der jeweiligen Reagentien in 5 Küvetten zur Bestimmung der Reagentien-Leerwerte |
| 3 | | | | | | |
| 23 | $\left(\dfrac{\Delta E}{min}\right)_1$ | $\left(\dfrac{\Delta E}{min}\right)_1$ | $\left(\dfrac{\Delta E}{min}\right)_1$ | $\Delta E_1$ | $\Delta E_1$ | |
| 43 | | | | | | |
| 63 | | | | | | |
|  | ✗ | ✗ | ✗ | ✗ | ✗ | Pipettierung von Probe in alle 5 Ansätze |
| 93 | | | | | | |
| 113 | | | | | | |
| 133 | | | | | | |
| 153 | | | | $\Delta E_2$ | $\Delta E_2$ | |
| 173 | | | | | | |
| 193 | $\left(\dfrac{\Delta E}{min}\right)_2$ | $\left(\dfrac{\Delta E}{min}\right)_2$ | | | | |
| 213 | | | | | | |
| 233 | | | | | | |
| 253 | | | | | | |
| 273 | | | | | | |
|  | | | | ✗ | ✗ | Pipettierung von Standards in Glucose- + Harnstoff-Ansatz |
| 303 | | | | | | |
| 323 | | | | $\Delta E_3$ | $\Delta E_3$ | |
| 343 | | | $\left(\dfrac{\Delta E}{min}\right)_2$ | | | |
| 363 | | | | | | |
| 383 | | | | | | |
| 403 | | | | | | |
| 423 | | | | | | |

GOT  GPT  CK  Glukose  Harn-stoff

Pipettierung der Kontrolle in alle 5
Ansätze

Zeitpunkt  0:

$\Delta E_4$   $\Delta E_4$

$\left(\dfrac{\Delta E}{min}\right)_3$   $\left(\dfrac{\Delta E}{min}\right)_3$

$\left(\dfrac{\Delta E}{min}\right)_3$

453
473
493
513
533
553
573
593
613
633
653
673
693
713
723
743

↑

sec

4. Messzyklus

– 10 –

0044388

Im ersten Messzyklus (z.B. 3-63 Sekunden) werden für alle fünf verschiedenen Bestimmungen die Reagenzien-Leerwerte ermittelt, dann wird Probe zu den fünf verschiedenen Reagenzien-Ansätzen pipettiert.

Im zweiten Messzyklus (z.B. 93-273 Sekunden) werden die zur Berechnung erforderlichen Extinktionen für den GOT-, GPT-, Glukose- und Harnstoff-Gehalt in der Probe gemessen. Bei der Bestimmung der Glukose und des Harnstoffes sind es 2-Punkt-Messungen (Endpunktbestimmung). Bei der GOT- und GPT-Bestimmung sind es mehrere Extinktionsmessungen im Abstand von z.B. 20 Sekunden (kinetische Bestimmung). Da die CK-Bestimmung - chemisch bedingt - eine Vorinkubationszeit benötigt, werden die zur Berechnung erforderlichen Extinktionen erst im 3. Messzyklus gemessen.

Im dritten Messzyklus (z.B. 303-423 Sekunden) werden nach Pipettierung von Standardlösung zu den Glukose- und Harnstoff-Ansätzen die zur Berechnung erforderlichen Extinktionen gemessen. Im 3. Messzyklus erfolgen auch die Extinktionsmessungen für die CK-Bestimmung. Daran anschliessend erfolgt die Pipettierung der Kontrollwerte in die fünf verschiedenen Bestimmungs-Ansätze und im 4. Messzyklus (z.B. 453-743 Sekunden) werden die zur Berechnung erforderlichen Extinktionen gemessen.

Die Berechnung erfolgt nach der allgemein angewandten und bekannten Regeln mit den Extinktionsdifferenzen pro Zeiteinheit bei den Enzymbestimmungen und mit den Extinktionsdifferenzen zwischen der Extinktion am Ende der Reaktion und der entsprechenden Extinktion für den Proben-Leerwert bei Substratbestimmungen.

Bei den in die Berechnungsformeln eingesetzten Extinktionen bzw. Extinktionsdifferenzen handelt es sich um die den unterschiedlichen Volumenverhältnissen Rechnung getragenen Extinktionen.

$$\left.\begin{array}{l} \text{GOT-} \\ \text{GPT-} \\ \text{CK-} \end{array}\right\} \text{Bestimmung:} \left\{\left(\frac{\Delta E}{\min}\right)_3 - \left(\frac{\Delta E}{\min}\right)_2\right\} \times F_E = \text{Aktivität in der Kontrollprobe}$$

$$\left\{\left(\frac{\Delta E}{\min}\right)_2 - \left(\frac{\Delta E}{\min}\right)_1\right\} \times F_E = \text{Aktivität der zu analysierenden Probe}$$

$$F_E = \text{theoretischer Faktor für die Enzymbestimmungen}$$

$$\left.\begin{array}{l} \text{Glucose-} \\ \text{Harnstoff-} \end{array}\right\} \text{Bestimmung:} \quad \text{Faktor zur Berechnung} = F_S = \frac{\text{Konzentration des Standardes}}{(\Delta E_3 - \Delta E_1)}$$

$$F_S = \text{Faktor für die Substratbestimmung}$$

$$\left\{\Delta E_4 - \Delta E_1\right\} \times F_S = \text{Gehalt in der Kontrollprobe}$$

$$\left\{\Delta E_3 - \Delta E_1\right\} \times F_S = \text{Gehalt in der zu analysierenden Probe}$$

Die nachfolgenden Beispiele erläutern die Erfindung.

## Beispiel 1

a) __Glukosebestimmung__ (manuell durchgeführte Bestimmung; insbesondere geeignet für Einzelbestimmung und für Notfallbestimmungen)
Methode: Glukoseoxidase, Peroxidase, p-Hydroxybenzoesäure und 4-Aminoantipyrin

In einer Küvette für Einmalgebrauch wird ein Granulatgemisch für 2 ml Reagens konfektioniert.

Zu dem Inhalt der Küvette werden 2 ml Wasser pipettiert und nach dem Auflösen des Reagentiengemisches und Einbringen der Küvette in das Photometer wird die Extinktion auf 0 gestellt ($\lambda$ = 546 nm, 37°C). Anschliessend werden 10 µl Probe zu dem Reaktionsgemisch pipettiert und nach 3 Minuten die Extinktion $E_1$ gemessen. Daraufhin werden 10 µl Standard pipettiert und nach ca. 1,5 Minuten die Extinktion $E_2$ gemessen. Zur Qualitätskontrolle werden nochmals zu dem Reaktionsgemisch 10 µl eines Kontrollserums für den Normalbereich pipettiert und nach ca. 1,5 Minuten wiederum die Extinktion gemessen (= $E_3$).

Diese Methode ist besonders geeignet für Schnellbestimmungen. Da mit 2 ml Reagens und nur jeweils mit 10 µl Probe gearbeitet wird und ausserdem bei 546 nm die Extinktion gemessen werden, erübrigen sich die speziellen Berücksichtigungen für Proben-Leerwerte.

Als unbekannte Probe werden Moni-Trol II ① (Deklaration: 232-__258__-284 mg/dl)
Standard 90 mg/dl und Hyland I ② (Deklaration: 68,5-__74,5__-80,5 mg/dl
eingesetzt.

---

① Kontrollserum der Firma Merz & Dade, Düdingen (CH) mit Werten im pathologischen Bereich
② Kontrollserum der Firma Hyland (Vertreter: Travenol Int. GmbH, 8 München 2) mit Werten im Normalbereich

Gemessene Extinktionen: $E_1$ = 0,300, $E_2$ = 0,407, $E_3$ = 0,492

Berechnung: $\Delta E_{Probe}$ = Moni-Trol II = 0,300

$\Delta E_{Standard}$ = 0,107

$\Delta E_{Kontrolle}$ = Hyland I = 0,085

Konzentration in der Probe  $c[mg/dl] = \dfrac{0,300}{0,107} \times 90 = 252$ mg/dl

im Kontrollserum:  $c[mg/dl] = \dfrac{0,085}{0,107} \times 90 = 71,5$ mg/dl

b)  <u>Glukosebestimmung</u> (auf Zentrifugalanalysator)
Methode: Hexokinase/Glukose-6-phosphat-Dehydrogenase

Unter Verwendung des Autoblanking-Verfahrens werden die Proben-Leerwerte, die durch die Eigenextinktion der Proben bedingt sind, berücksichtigt.

Zu 200 µl Reagens werden 5 µl Probe pipettiert. Nach Beendigung der Reaktion (5 Minuten) werden nochmals zu dem gleichen Ansatz 5 µl Probe pipettiert und aus den erhaltenen Extinktionsdifferenzen die Konzentration bestimmt.

| Kontrollserum | Deklarations-wert | gefundener Wert | gefundener Wert für das Kontrollserum bei nochmaliger Pipettierung von 5 µl Probe |
|---|---|---|---|
| Moni-Trol I [1] | 86-<u>96</u>-106 | 97,1 | 96,9 |
| Moni-Trol II | 226-<u>246</u>-266 | 245,7 | 242,6 |
| Precipath U [2] | 198-<u>211</u>-224 | 205,2 | 212,0 |

c)  <u>Glukosebestimmung</u> (auf Zentrifugalanalysator)
Methode: Hexokinase/Glukose-6-phosphat-Dehydrogenase

---

[1] Kontrollserum der Firma Merz & Dade, Düdingen (CH) mit Werten im Normalbereich

[2] Kontrollserum der Firma Böhringer Mannheim mit Werten im pathologischen Bereich

Die Proben-Leerwerte werden durch das Autoblanking-Verfahren berücksichtigt. Zu 250 µl Reagenz werden jeweils 5 µl Patientenprobe pipettiert und die auftretenden Extinktionsdifferenzen nach einer Reaktionszeit von 5 Minuten gemessen. Anschliessend werden zur Kontrolle der Linearität und für die Zuverlässigkeitskontrolle nochmals 5 µl eines Kontrollserums pipettiert und wiederum die Extinktionsdifferenzen gemessen. Die Berechnung erfolgt bei diesem Beispiel faktoriell.

Patientenwert     Kontrollserumwert (Moni Trol), Deklarationswert: 86-96-106

| Patientenwert | Kontrollserumwert | |
|---|---|---|
| 74,5 | 90,9 | |
| 135,1 | 89,4 | |
| 126,5 | 92,2 | |
| 107,1 | 90,3 | |
| 130,4 | 91,1 | |
| 392,4 | 91,1 | |
| 430,6 | 84 | * |
| 527,7 | 33,8 | ** |

\*    Anzeige, dass an Grenze des Linearitätsbereiches

\*\*   Anzeige, dass Linearitätsbereich überschritten

Dieses Beispiel zeigt, dass durch Betrachtung des jeweiligen Konrollserumwertes, Aussagen gemacht werden können über photometrische Begrenzung, Begrenzung an einer Komponente im Reagens, an einer ev. nicht ausreichender Reaktionszeit. In letzterem Fall käme - innerhalb des Linearitätsbereiches - für das Kontrollserum ein zu hoher Wert heraus.

<u>d)</u>   <u>Glukosebestimmung</u> (manuell durchgeführte photometrische Bestimmung) Methode: Hexokinase/Glukose-6-phosphat-Dehydrogenase

Zu 1000 µl Reagens werden 20 µl Probe (eingesetzt wird ein Kontrollserum; Lab Trol)① pipettiert und nach 5 Minuten die Extinktion $E_1$ gemessen, dann werden zu dem gleichen

---

① Kontrollserum der Firma Merz & Dade, Düdingen (CH) mit Werten im Normalbereich

Ansatz 20 µl einer Lösung (Standard) von 100 mg/dl hinzupipettiert und nach weiteren 2 Minuten die Extinktion $E_2$ gemessen. Zur Kontrolle des Linearitätsbereiches, der Standardpipettierung und als Gesamtkontrolle für das System: Apparat, Reagenzien und Berechnung werden zusätzlich noch 20 µl eines Kontrollserums hinzupipettiert und nach weiteren 2 Minuten $E_3$ gemessen.

|  |  | Endvolumen |
|---|---|---|
| $E_1$: | 0,282 | 1,020 |
| $E_2$: | 0,638 | 1,040 |
| $E_3$: | 0,991 | 1,060 |

Unter Einsatz von nur 1 x Reagenz werden hintereinander die für eine Bestimmung erforderlichen 3 Ansätze: Probenansatz, Standardansatz zur Berechnung und Kontrollserumansatz für die Zuverlässigkeitsüberwachung durchgeführt.

Beispiel 2

a) GOT-Bestimmung

Die GOT-Bestimmung erfolgt auf einem COBAS-BIO Zentrifugalanalysator. Bei dieser Bestimmung werden hintereinander drei verschiedene Kontrollseren pipettiert und die jeweiligen Extinktionsdifferenzen und die den Extinktionsdifferenzen entsprechenden Aktivitäten ermittelt.

Zu 250 µl Reagens werden zuerst 10 µl Hyland I (deklarierte Werte): 19,5-23,2 - 26,9 U/l) dann 10 µl Roche-Enzym-Kontrollserum N[1](deklarierte Werte der verwendeten Charge X 0242: 33-39 - 45 U/l) und dann 10 µl Hyland II[2](deklarierte Werte: 74-79 - 83 U/l) pipettiert.

---

[1] Enzym Kontrollserum von F.Hoffmann-La Roche & Co. AG, Diagnostica, CH-4002 Basel mit Werten im Normalbereich

[2] Kontrollserum der Firma Hyland (Vertreter: Travenol Int. GmbH 8 München 2) mit Werten im pathologischen Bereich

Die erhaltenen Aktivitäten nach der ersten Pipettierung waren: A1 = 22,7 U/l, nach der zweiten Pipettierung: A2 = 56,8 U/l und nach der dritten Pipettierung A3 = 132,4 U/l.

gefundene Aktivität für Hyland II                     : $A_3-A_2$ = 75.6 U/l

gefundene Aktivität für Roche Enzym Kontrollserum N: $A_2-A_1$ = 34.1 U/l

gefundene Aktivität für Hyland I                      : $A_1$


b)    GOT-Bestimmung in Patientenseren


Bei der Bestätigung der Durchführbarkeit der Transaminase-Bestimmung GOT wurde folgendermassen vorgegangen:

Zu je 150 µl GOT-Reagens werden 15 µl Patientenserum pipettiert und normal die Aktivität aus den gemessenen Extinktionsdifferenzen pro Minute berechnet (= Aktivität I). Danach wird jeweils zu allen Reaktionslösungen nochmals 15 µl eines Kontrollserums pipettiert und aus den erneut gemessenen Extinktionsmessungen beider Proben (Patientenprobe + Kontrollserumprobe) die Aktivität (= Aktivität II) berechnet. Aus der Differenz: Aktivität II - Aktivität I wird der Kontrollserumwert berechnet. Liegt der erhaltene Aktivitätswert für das Kontrollserum im Deklarationsbereich, liegen folgende Bestätigungen vor: 1. Alle Aussagen bezüglich der normalen Qualitätskontrolle und 2. die Aussage, dass der Linearitätsbereich bei keiner Patientenprobe überschritten wurde. Andernfalls wäre der entsprechende Kontrollserumwert zu tief herausgekommen. Weiterhin kann aus diesem Beispiel ersehen werden, dass die 2. Aktivitätsbestimmung - wie aus dem gleichen Wert (innerhalb der allgemeinen Fehlergrenze) für das Kontrollserum ersehen werden kann - nicht durch Begleitstoffe in den Patientenproben beeinflusst wird.


Die Resultate können den nachfolgenden Tabellen entnommen werden:

## Tabelle

Ermittlung der Transaminase-Aktivität GOT
Aktivität I: GOT-Aktivität der Patientenprobe
Aktivität II: GOT-Aktivität der Patientenprobe + des Kontrollserums
Aktivität II - Aktivität I: Erhaltene GOT-Aktivität des
Kontrollserums

| Patienten-Nr. der eingesetzten Seren | A. Zusatz von Enzym-Control-Serum N | | |
| --- | --- | --- | --- |
| | Aktiv. I U/l | Aktiv. II U/l | Aktiv. II - Aktiv. I U/l. |
| 1054 | 136,55 | 185,78 | 49,23 |
| 1083 | 128,62 | 178,28 | 49,66 |
| 1093 | .88,78 | 140,60 | 51,82 |
| 1126 | 51,60 | 102,91 | 51,31 |
| 1076 | 108,55 | 161,81 | 53,26 |
| 1173 | 44,42 | 98,73 | 54,31 |
| 1136 | 42,75 | 95,23 | 52,48 |
| 1131 | 34,82 | 87,16 | 52,34 |
| 1028 | 146,20 | 198,05 | 51,85 |
| 1011 | 37,55 | 90,53 | 52,98 |
| 1010 | 104,02 | 155,85 | 51,83 |
| 1265 | 83,71 | 130,76 | (47,05) |
| 1004 | 41,74 | 96,01 | 54,27 |
| 1178 | 59,27 | 114,50 | 55,23 |
| 1002 | 107,79 | 160,14 | 52,35 |
| 1081 | 118,77 | 171,88 | 53,11 |
| 1173 | 42,51 | 93,47 | 50,96 |
| 1092 | 81,25 | 133,50 | 52,25 |
| 1243 | 8,86 | 61,63 | 52,77 |
| 1074 | 44,56 | 96,38 | 51,82 |
| Enzym-Kontroll-serum ① | 50,23 50,14 | 102,69 102,78 | 52,46 52,64 |
| Monitrol II | 36,25 37,11 | 87,75 87,09 | 51,50 49,98 |
| Precinorm U ② | 27,33 | 78,92 | 51,59 |

Deklarationswert für Enzym-Kontrollserum N 48-53-58

gefundener Mittelwert: $\bar{x} = 51,96$
tiefster Wert:             $= 49,23$
höchster Wert:            $= 55,23$

$s = 1,7107$
$VK = 3,29$

① Enzym Kontrollserum der F. Hoffmann-La Roche & Co. AG,
Diagnostica, CH-4002 Basel, mit Werten im Normalbereich
② Kontrollserum der Firma Böhringer Mannheim mit Werten im
Normalbereich

Tabelle (Fortsetzung)

Ermittlung der Transaminase-Aktivität GOT
Aktivität I: GOT-Aktivität der Patientenprobe
Aktivität II: GOT-Aktivität der Patientenprobe + des Kontrollserums
Aktivität II - Aktivität I: Erhaltene GOT-Aktivität des
Kontrollserums

| Patienten-Nr. der eingesetzten Seren | B. Zusatz von Precinorm U | | |
| --- | --- | --- | --- |
| | Aktiv. I<br><br>U/l | Aktiv. II<br><br>U/l | Aktiv. II<br>-<br>Aktiv. I<br>U/l |
| 1054 | 137,23 | 167,39 | 30,16 |
| 1083 | 130,62 | 159,36 | 28,74 |
| 1093 | 90,55 | 118,03 | 27,48 |
| 1126 | 51,25 | 80,08 | 28,83 |
| 1076 | 108,76 | 140,57 | 31,81 |
| 1173 | 45,35 | 71,46 | 26,11 |
| 1136 | 41,89 | 69,43 | 27,54 |
| 1131 | 35,57 | 60,99 | 25,42 |
| 1028 | 145,02 | 179,32 | (34,30) |
| 1011 | 39,49 | 65,44 | 25,95 |
| 1010 | 105,87 | 136,33 | 30,46 |
| 1265 | 84,60 | 112,64 | 28,04 |
| 1004 | 43,44 | 70,84 | 27,40 |
| 1178 | 59,87 | 89,32 | 29,45 |
| 1002 | 109,79 | 138,89 | 29,10 |
| 1081 | 119,87 | 147,19 | 27,32 |
| 1173 | 41,08 | 67,50 | 26,42 |
| 1092 | 81,88 | 108,08 | 26,20 |
| 1243 | 8,59 | 36,99 | 28,40 |
| 1074 | 42,16 | 70,73 | 28,57 |
| Enzym-Kontroll-serum ① | 50,74<br>52,30 | 80,30<br>80,11 | 29,56<br>27,81 |
| Monitrol II | 36,85<br>35,71 | 69,58<br>68,35 | 32,73<br>32,64 |
| Precinorm U ② | 26,87 | 55,88 | 29,01 |

Deklarationswert für Precinorm U
25-29-33

gefundener Mittelwert: $\bar{X}$ = 28,78
tiefster Wert:           = 26,11
höchster Wert:           = 32,73
                       s = 2,2702
                      VK = 7,89

① Enzym Kontrollserum der F.Hoffmann-La Roche & AG,.
Diagnostica, CH-4002 Basel, mit Werten im Normalbereich
② Kontrollserum der Firma Böhringer Mannheim mit Werten im
Normalbereich

Tabelle (Fortsetzung)

Ermittlung der Transaminase-Aktivität GOT
Aktivität I: GOT-Aktivität der Patientenprobe
Aktivität II: GOT-Aktivität der Patientenprobe + des Kontroll-serums
Aktivität II – Aktivität I: Erhaltene GOT-Aktivität des Kontrollserums

| Patienten-Nr. der eingesetzten Seren | C. Zusatz von Monitrol II | | |
| --- | --- | --- | --- |
| | Aktiv. I U/l | Aktiv. II U/l | Aktiv. II – Aktiv. I U/l |
| 1054 | 137,20 | 174,06 | 36,86 |
| 1083 | 128,37 | 163,58 | 35,21 |
| 1093 | 92,53 | 128,55 | 36,02 |
| 1126 | 51,71 | 86,98 | 35,27 |
| 1076 | 109,91 | 141,75 | (31,84) |
| 1173 | 44,07 | 80,13 | 36,06 |
| 1136 | 40,57 | 78,55 | 37,98 |
| 1131 | 33,69 | 70,94 | 37,25 |
| 1028 | 145,51 | 184,18 | 38,67 |
| 1011 | 36,44 | 74,23 | 37,79 |
| 1010 | 104,71 | 142,00 | 37,29 |
| 1265 | 82,51 | 119,39 | 36,88 |
| 1004 | 41,40 | 79,76 | 38,36 |
| 1178 | 60,79 | 99,90 | 39,11 |
| 1002 | 109,61 | 145,61 | 36,00 |
| 1081 | 119,70 | 157,67 | 37,97 |
| 1173 | 40,26 | 76,75 | 36,49 |
| 1092 | 82,43 | 119,29 | 36,86 |
| 1243 | 8,12 | 47,71 | 39,59 |
| 1074 | 41,89 | 81,12 | 39,23 |
| Enzym-Kontroll serum ① | 49,95 50,82 | 88,49 89,29 | 38,54 38,47 |
| Monitrol II | 36,27 35,86 | 71,88 73,15 | 35,61 37,29 |
| Precinorm U ② | 26,09 | 67,16 | 41,07 |

Deklarationswert für Monitrol II
35–43–50

gefundener Mittelwert: $\bar{x}$ = 37,27
tiefster Wert: = 35,21
höchster Wert: = 41,07
s = 1,8341
VK = 4,92

① Enzym Kontrollserum der F.Hoffmann-La Roche & Co. AG, Diagnostica, CH-4002 Basel, mit Werten im Normalbereich
② Kontrollserum der Firma Böhringer Mannheim mit Werten im Normalbereich

## Beispiel 3

Cholesterinbestimmung

Methode: Cholesterinesterase/Cholesterinoxidase/ Peroxidase (PAP-Reaktion)

Zu 250 µl Reagens werden 5 µl Probe pipettiert und aus der erhaltenen Extinktionsdifferenz der Gehalt bestimmt. Nach jeweiliger Beendigung der Reaktionszeit werden nochmals 2 x hintereinander je 5 µl Probe pipettiert und aus den auftretenden Extinktionsdifferenzen der Gehalt berechnet.

| Kontrollserum (als Probe eingesetzt) | Deklarations- bereich | gefundener Wert | ermittelter Gehalt nach weiterer Zu- pipettierung von | |
|---|---|---|---|---|
| | | | + 5 µl | + 5 µl |
| Moni-Trol I | 111-126-141 | 121,9 | 121,6 | 127,6 |
| Moni-Trol II | 188-213-238 | 217,6 | 202,3 | 209,6 |
| Precinorm U | 118-131-144 | 119 | 119,2 | 119,3 |

## Beispiel 4

Bestimmung der Transaminase GPT in Patientenseren

Bei der Bestätigung der Durchführbarkeit der Transaminase-Bestimmung GPT wird folgendermassen vorgegangen: Zu je 100 µl GPT-Reagens werden 10 µl Patientenserum pipettiert und normal die Aktivität aus den gemessenen Extinktionsdifferenzen pro Minute berechnet (= Aktivität I). Danach wird jeweils zu allen Reaktionslösungen nochmals 10 µl eines Kontrollserums pipettiert und aus den erneut gemessenen Extinktionsmessungen beider Proben (Patientenprobe + Kontrollserumprobe) die Aktivität (= Aktivität II) berechnet. Aus der Differenz: Aktivität II - Aktivität I wird der Kontrollserumwert berechnet.

| Patienten-Nr. der eingesetzten Seren | Aktivität I U/l | Aktivität II U/l (nach Zusatz von 10 µl eines Kontrollserums) | Δ |
|---|---|---|---|
| 1132 | 81,8 | 132,7 | 50,9 |
| 1277 | 38,4 | 90,9 | 52,5 |
| 1033 | 75,3 | 125,1 | 49,8 |
| 1308 | 92,6 | 143,8 | 51,2 |
| 1080 ikterisch | 51,3 | 102,7 | 51,4 |
| 1013 ikterisch | 1359,0 | – | – |
| 1128 | 35,9 | 88,5 | 52,6 |
| 1075 | 198,1 | 249,6 | 51,5 |
| 1078 ikterisch | 39,4 | 91,7 | 52,3 |
| 1235 lipämisch | 38,2 | 88,2 | 50,0 |
| 1327 lipämisch | 28,3 | 77,9 | 49,6 |
| 1264 | 29,1 | 81,0 | 51,9 |

$\bar{x} = 51,2$, s = 1,1

Deklarationsbereich mit Berücksichtigung eines kinetischen Reagentien-Leerwertes: 49-<u>53</u>-57

Patentansprüche

1. Verfahren zur Bestimmung von einem oder mehreren Parametern in Proben, dadurch gekennzeichnet, dass pro Parameter in ein und derselben Messzelle und mit ein und demselben Reagens mehrere gleiche Bestimmungen hintereinander und nur mit einmaliger Pipettierung von Reagens durchgeführt werden.

2. Verfahren nach Anspruch 1, dadurch gekennzeichnet, dass die einzelnen Bestimmungen, die mit demselben Reagens hintereinander durchgeführt werden, mehrere verschiedene Proben und/oder Standard-Ansätze betreffen, die in die Messzelle nacheinander eingeführt werden.

3. Verfahren nach Anspruch 1, dadurch gekennzeichnet, dass die einzelnen Bestimmungen, die mit demselben Reagens hintereinander durchgeführt werden, einen Regenzien-Leerwert-Ansatz, mindestens einen Probenansatz, einen Standard-Ansatz und einen Kontrollserum-Ansatz betreffen, welche in die Messzelle nacheinander eingeführt werden.

4. Verfahren nach Anspruch 1, dadurch gekennzeichnet, dass man in mehreren verschiedenen Proben einen Parameter bestimmt.

5. Verfahren nach Anspruch 1, dadurch gekennzeichnet, dass man in mehreren gleichen Proben mehrere Parameter bestimmt.

\*\*\*